# EUROPÄISCHE PATENTSCHRIFT

(11) **EP 1 136 094 B1**
(45) Veröffentlichungstag und Bekanntmachung des Hinweises auf die Patenterteilung: **26.04.2006**
(21) Anmeldenummer: 01106851.7
(22) Anmeldetag: 19.03.2001
(51) Int. Cl.: A61M 16/00

(54) **Beatmungsgerät mit einer Überwachungsvorrichtung**
Ventilator with monitoring device
Appareil de ventilation artificielle avec un dispositif de surveillance

(30) Priorität: 24.03.2000 DE 10014427
(43) Veröffentlichungstag der Anmeldung: 26.09.2001
(73) Patentinhaber: Weinmann Geräte für Medizin GmbH & Co. KG, 22525 Hamburg (DE)
(72) Erfinder: Schöller, Bernd, 76135 Karlsruhe (DE); Graetz, Bernd, 22869 Schenefeld (DE); Maurer, Jörg, 21521 Auhmühle (DE)
(74) Vertreter: Klickow, Hans-Henning

(56) Entgegenhaltungen:
- EP-A- 0 691 134
- US-A- 5 803 066
- FARRE R. ET AL: 'SERVOCONTROLLED GENERATOR TO MEASURE RESPIRATORY IMPEDANCE FROM 0.25 TO 26 HZ IN VENTILATED PATIENTS AT DIFFERENT PEEP LEVELS' EUROPEAN RESPIRATORY JOURNAL Bd. 8, Nr. 7, Juli 1995, DENMARK, Seiten 1222 - 1227, XP001203575
- PESLIN R. ET AL: 'RESPIRATORY MECHANICS STUDIED BY FORCED OSCILLATIONS DURING ARTIFICIAL VENTILATION' EUROPEAN RESPIRATORY JOURNAL Bd. 6, Nr. 6, 1993, DENMARK, Seiten 772 - 784, XP009039367

## Beschreibung

Die Erfindung betrifft ein Beatmungsgerät, in dem mindestens zwei unterschiedliche Druckpegel für eine Atemgasversorgung einstellbar sind und bei dem mindestens ein Beatmungsparameter meßtechnisch erfaßt und zur Steuerung des Beatmungsdruckes ausgewertet wird, sowie bei dem mindestens einer der Beatmungsparameter in Abhängigkeit von einer Mustererkennung verändert wird, wobei zur Durchführung der Mustererkennung wenigstens intervallweise der zeitliche Verlauf mindestens eines Beatmungsparameters erfaßt und im Hinblick auf typische Verlaufsmuster analysiert wird, sowie bei dem eine Auswertung sowohl des Beatmungsdruckes als auch des Beatmungsflows erfolgt.

Das Beatmungsgerät ist mit einer Vorrichtung zur Überwachung mindestens eines Beatmungsparameters bei der Atemgasversorgung eines Patienten versehen, die mindestens einen Sensor zur Erfassung eines zeitlichen Verlaufes des Beatmungsparameters aufweist, der im Bereich einer Luftzuführung angeordnet ist.

Eine nicht unbeachtliche Anzahl von Menschen leiden unter Schlafstörungen, die sich auf das Tagesbefinden dieser Menschen auswirken und deren soziale und berufliche Leistungsfähigkeit sowie deren Lebensqualität zum Teil erheblich beeinträchtigen. Eine dieser schlafstörungen ist die Schlafapnoe, die vorrangig mit der sogenannten CPAP-Therapie (CPAP = Continuous Positive Airway Pressure) behandelt wird, indem dem Patienten während des Schlafes ein Luftstrom aus einem atemfähigen Gas über eine Nasalmaske kontinuierlich zugeführt wird. Die Maske ist über einen Schlauch mit einem Beatmungsgerät verbunden, das einen Lüfter umfaßt, der einen Gasstrom mit einem Überdruck von 5 bis 20mbar erzeugt.

Der Gasstrom wird dem Patienten entweder mit konstantem Druck zugeführt, oder zur Erleichterung der Atemarbeit des Patienten beim Ausatmen auf ein niedrigeres Druckniveau abgesenkt. Obwohl Schlafapnoen nur kurzfristig auftreten und einen geringen Teil des Schlafes ausmachen, läuft der Lüfter bei beiden verfahren während der gesamten Schlafdauer (Nacht), was die Akzeptanz dieser Schlafapnoe-Behandlung erschwert.

Aus der US-A-5 245 995 ist ein CPAP-Beatmungsgerät bekannt, das für Patienten mit Schlafapnoe einsetzbar ist. Das Atemgas wird dem Patienten über eine Atemmaske zugeführt und im Bereich des Gerätes ist eine Druckgasquelle angeordnet. Die Druckgasquelle ist in Abhängigkeit vom Atemwegswiderstand steuerbar.

Aus der EP-A-0 373 585 ist es bekannt, den Atemwiderstand eines Patienten mit Hilfe der ORM-Messung zu erfassen. Mit vorgebbarer Frequenz wird hierbei dem Atemvolumenstrom ein oszillierender Volumenstrom mit geringem Volumenhub überlagert. Aufgrund der mit gleicher Frequenz auftretenden periodischen Druckschwankung kann ein vom konkreten Atemwegswiderstand abhängiger Meßwert bereitgestellt werden.

Aus der US-A-5 318 038 ist es bekannt, eine Atemmessung unter Verwendung eines Pneumottachographen in der Gaszuführleitung durchzuführen. Die EP-A-0 705 615 beschreibt eine qualitativ hochwertige Realisierung einer Steuerung eines Beatmungsgerätes auf Grund der Durchführung von ORM-Messungen.

Aus der US-A-5,803,066 ist es bekannt, ein Beatmungsgerät mit einer Steuerung zu koppeln, der von einem Drucksensor sowie von einem Flowsensor erfaßte Meßwerte zugeführt werden. Die Meßwerte werden in Bereich einer verbindungsleitung zwischen dem Beatmungsgerät und einer Atemmaske des Patienten erfaßt. Die Steuerung umfaßt eine Meßdatenverarbeitung, um eine verbesserte Ansteuerung des Beatmungsgerätes im Rahmen einer CPAP-Therapie zu ermöglichen. Signalverläufe des Druckes und des Flows werden hierbei von einem Signalprozessor ausgewertet, der die jeweiligen Signalverläufe hinsichtlich des Überschreitens von Grenzwerten überwacht. Durch eine entsprechende Signalauswertung ist es möglich, unterschiedliche Atmungstypen zu unterscheiden.

Aus der EP 0 691 134 ist ein Beatmungsgerät bekannt, bei dem unter Berücksichtigung einer meßtechnisch erfaßten Flowimpedanz ein Druck ermittelt wird, der erforderlich ist, um die Lunge eines Patienten pro Atemzug mit einem vorgegebenen Gasvolumen zu füllen. In Abhängigkeit von der Auswertung der Flowimpedanz erfolgt eine ventilumschaltung, um Inspirationsphasen und Expirationsphasen durchzuführen.

Aus der EUROPEAN RESPIRATORY JOURNAL, Bd. 8, Nr. 7, Juli 1995 (1995-07), Seiten 1222-1227, XP001203575 DENMARK ist es bekannt, einen servogesteuerten Generator zur Messung der inspiratorischen Impedanz der Atemwege eines Patienten zu verwenden. Es liegt ein typischer Arbeitsbereich von 0,25 Hz bis 26 Hz vor.

Aus der EUROPEAN RESPIRATORY JOURNAL, Bd. 6, Nr. 6, 1993, Seiten 772-784, XP009039367 DENMARK ist ebenfalls die Bestimmung der respiratorischen Mechanik der Atemwege eines Patienten durch Aufbringung von erzwungenen Oszillationen des Atemgases bekannt. Die erforderliche Anregungsschwingung kann unter Verwendung einer Lautsprechermembran aufgebracht werden. Als Meßergebnis liegt die respiratorische Impedanz der Atemwege vor.

Weder im Zusammenhang mit einer direkten meßtechnischen Erfassung vom Druck und Flow noch im Zusammenhang mit einer indirekten Messung von Druck und/oder Flow durch Erfassung zu diesen Größen proportionaler Meßgrößen wird aber die zusätzliche Auswertung eines ODS-Signals zur Ermittlung der respiratorischen Impedanz im Stand der Technik beschrieben, um ein Beatmungsgerät zu steuern.

Die Verfahren und Vorrichtungen gemäß dem Stand der Technik ermöglichen es bereits mit sehr hoher Sicherheit, lebensbedrohliche Zustände des Patienten auszuschließen. Es können jedoch bislang noch nicht alle Anforderungen an eine möglichst geringe Beeinträchtigung der allgemeinen Lebensumstände des Patienten erfüllt werden. Ein besonderes Ziel besteht darin, die eigene Atmungsaktivität des Patienten möglichst unverändert zu lassen, und eine gerätetechnische Unterstützung nur dann vorzunehmen, wenn dies auch tatsächlich erforderlich ist. Ein derartiges Ziel erfordert eine Optimierung der Steuerung und Regelung des Beatmungsgerätes sowie eine Bereitstellung geeigneter regelungstechnischer Komponenten. Insbesondere ist es erforderlich, möglichst frühzeitig Abweichungen von der normalen Atmungstätigkeit des Patienten zu erkennen und hierauf durch eine geeignete Steuerung und Regelung des Atmungsgerätes zu reagieren.

Aufgabe der vorliegenden Erfindung ist es daher, ein Beatmungsgerät der einleitend genannten Art derart zu verbessern, daß eine frühzeitige Adaption der Gerätefunktion an einen jeweiligen Beatmungszustand unterstützt wird.

Diese Aufgabe wird erfindungsgemäß dadurch gelöst, daß zusätzlich zur Auswertung des Beatmungsdruckes und des Beatmungsflows eine Auswertung einer aus einem ODS-Signal ermittelten respiratorischen Impedanz durchgeführt wird und daß im Anschluß an die Mustererkennung eine Klassenzuordnung durchgeführt wird.

Weitere Aufgabe der vorliegenden Erfindung ist es, eine Vorrichtung der einleitend genannten Art derart zu konstruieren, daß bei einfachem gerätetechnischen Aufbau frühzeitige Änderungen einer jeweiligen Beatmungssituation erkannt werden.

Diese Aufgabe wird dadurch erfindungsgemäß gelöst, daß der Sensor mit einem Analysator verbunden ist, der eine Mustererkennung durchführt und der an eine Steuerung zur Veränderung mindestens eines Beatmungsparameters angeschlossen ist, sowie bei der der Analysator sowohl zur Auswertung eines Beatmungsdruckes als auch zur Auswertung eines Beatmungsflows ausgebildet ist und bei der der Analysator zusätzlich zur Auswertung des Beatmungsdruckes und des Beatmungsflows zur Auswertung einer aus einem ODS-Signal ermittelten respiratorischen Impedanz ausgebildet ist und daß der Analysator mit einem Klassifizierer gekoppelt ist.

Die Durchführung einer Mustererkennung bietet die Möglichkeit, sehr frühzeitig Änderungen eines aktuellen Atmungszustandes zu detektieren und hierdurch auch rechtzeitig eine veränderte Steuerung des Beatmungsgerätes durchzuführen. Es wird hierbei ausgenutzt, daß eine Vielzahl von Beatmungszuständen durch typische Verlaufsmuster, beispielsweise des Beatmungsdruckes oder des Beatmungsflusses, oder dazu proportionaler Größen, gekennzeichnet sind. Ebenfalls treten bereits relativ frühzeitig bei einem Übergang von einem Beatmungszustand zu einem anderen typische Verlaufsmuster auf.

Der Einsatz einer Mustererkennung sowie die frühzeitige Durchführung einer Veränderung der Gerätesteuerung ermöglicht es, bereits vor einer für den Patienten überhaupt bemerkbaren tatsächlichen Veränderung des Beatmungszustandes dieser Veränderung durch eine veränderte Gerätesteuerung derart entgegenzuwirken, daß zum einen kein für den Patienten gesundheitlich nachträglicher Beatmungszustand erreicht wird und daß andererseits die Gegenmaßnahmen so frühzeitig eingeleitet werden, daß bereits relativ geringe Veränderungen der Beatmungsparameter ausreichend sind, um den angestrebten Normalzustand wieder zu erreichen oder zumindest Abweichungen von diesem Normalzustand innerhalb eines sehr engen Abweichungsintervalls zu halten. Die frühzeitige Einleitung der Maßnahmen ermöglicht es, die Intensität der Gegenmaßnahmen sehr gering und für den Patienten in der Regel nicht bemerkbar zu halten.

Zur Erleichterung einer Detektierbarkeit signifikanter Muster wird vorgeschlagen, daß einem jeweiligen Druckpegel zur Atmungsunterstützung mindestens zeitweilig eine mit definierter Frequenz oszillierende Anregungsströmung überlagert wird. Dies umfaßt insbesondere auch eine Änderung der betreffenden Frequenz während der Durchführung der Therapie.

Ebenfalls trägt es zu einer vom Patienten als angenehm empfundenen Gerätesteuerung bei, daß nach einer selektiven Auswertung einer mit der Frequenz der Anregungsströmung auftretenden oszillatorischen Druckamplitude in der Luftzuführung eines Patienten, die zu einem Atemwiderstand des Patienten korrespondiert, eine Auswahl der jeweiligen Druckamplitude durchgeführt wird.

Eine typische Anwendung besteht darin, daß eine CPAP-Beatmung durchgeführt wird.

Eine Klasse auswertbarer Signale wird dadurch definiert, daß mindestens ein elektrisches Signal bei der Mustererkennung ausgewertet wird.

Ebenfalls ist daran gedacht, daß ein physikalisches Signal bei der Mustererkennung ausgewertet wird. Das physikalische Signal kann zur weiteren Verarbeitung in ein elektrisches Signal umgesetzt werden.

Zur Unterstützung einer systematischen Auswertung der Mustererkennung wird vorgeschlagen, daß im Rahmen der Mustererkennung eine Ableitung von Fehlerklassen durchgeführt wird.

Ein typischer auszuwertender Signalverlauf wird dadurch definiert, daß ein ODS-Signal ausgewertet wird. Unter ODS-Signal ist hierbei ein Oszillierendes-Druck-Signal zu verstehen, daß dem Betragswert der Impedanz entspricht.

Ebenfalls ist es möglich, daß ein Druck-Signal und/oder ein Schalldruck und/oder eine Druckschwankung ausgewertet wird.

Aufgrund der Rückwirkung der Erzeugung eines jeweiligen Beatmungszustandes auf die elektrischen Antriebsbedingungen der Druckgasversorgung ist auch daran gedacht, daß ein elektrischer Antriebsparameter der Druckgasversorgung ausgewertet wird. Dies erweist sich insbesondere als zweckmäßig, wenn eine Regelung über einen elektrischen Antrieb erfolgt. Alternativ kann die Regelung aber auch vom druckaufbauenden Antrieb unabhängig realisiert werden, beispielsweise über ein Ventil.

Gemäß einem typischen Auswertungsablauf ist vorgesehen, daß bei der Mustererkennung Formmerkmale ausgewertet werden.

Ebenfalls ist daran gedacht, daß bei der Mustererkennung Zeitmerkmale und/oder Amplitudenmerkmale ausgewertet werden.

Eine Aktivierung vorab definierter Steuerungsabläufe in Abhängigkeit von einem Ergebnis der Mustererkennung wird dadurch unterstützt, daß im Anschluß an die Mustererkennung eine Klassenzuordnung durchgeführt wird.

Zur Erhöhung der Auswertungsgeschwindigkeit ist vorgesehen, daß der Analysator mit einem Speicher zur Bereitstellung von Vergleichsmustern gekoppelt ist.

Ebenfalls trägt es zu einer hohen Verarbeitungsgeschwindigkeit bei, daß der Analysator mit einem Klassifizierer gekoppelt ist.

Eine kurzfristige Auswertung von Zeitverlaufscharakteristika der auszuwertenden Muster wird dadurch erleichtert, daß der Analysator einen Zeitverlaufsanalysator und/oder einen Amplitudenverlaufsanalysator aufweist.

Eine kurzfristige Auswertung von Formverlaufscharakteristika der auszuwertenden Muster wird dadurch erleichtert, daß der Analysator einen Formanalysator aufweist.

In der Zeichnung sind Ausführungsbeispiele der Erfindung schematisch dargestellt. Es zeigen:
- Fig. 1: ein prinzipielles Blockschaltbild zur Durchführung einer CPAP-Beatmung mit zusätzlicher Mustererkennung,
- Fig. 2: eine schematische Darstellung der wesentlichen Komponenten bei einer Durchführung einer CPAP-Beatmung,
- Fig. 3: eine Darstellung typischer Verlaufsmuster, auf die eine Mustererkennung anwendbar ist,
- Fig. 4: eine Darstellung von Meßsignalen bei einer Erfassung von Formmerkmalen bei einem Auftreten von Schnarchen und
- Fig. 5: eine Darstellung ähnlich zur Darstellung in Fig. 4 bei einer Erfassung von Zeitmerkmalen.

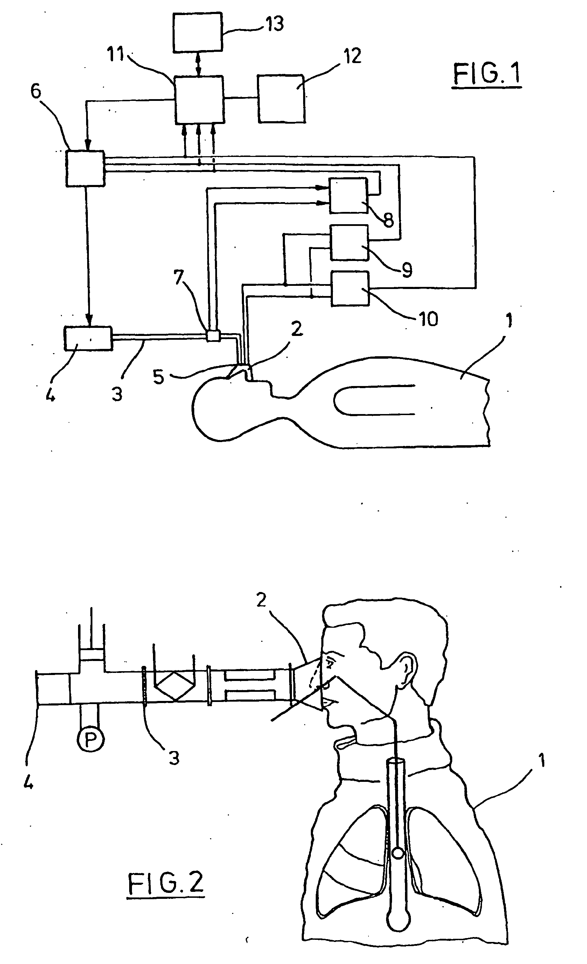

Fig. 1 zeigt in einer schematisierten Prinzipdarstellung einer Vorrichtung zur Steuerung eines Beatmungsgerätes. Das Beatmungsgerät ist hier zur CPAP-Beatmung eines Patienten (1) vorgesehen. Der Patient (1) ist über eine Atemmaske (2) und einen Atemschlauch (3) mit einer Druckgasquelle (4) verbunden. Die Druckgasquelle (4) kann beispielsweise als ein steuerbarer Lüfter realisiert sein. Bei der dargestellten Ausführungsform sind im Bereich der Atemmaske (2) ein oder mehrere Sensoren (5) angeordnet, um mindestens einen Beatmungsparameter zu erfassen. Gemäß einer anderen Ausführungsform können die Sensoren (5) aber auch im Bereich des Atemschlauches oder im Bereich der Druckgasquelle (4) angeordnet werden.

Zur Steuerung der Druckgasquelle (4) und zur Vorgabe von Beatmungsparametern, beispielsweise des Beatmungsdruckes und des Beatmungsflusses, ist die Druckgasquelle (4) mit einer Steuerung (6) verbunden. Zur Unterstützung einer Regelung eines Atemflusses ist insbesondere daran gedacht, im Bereich des Atemschlauches (3) eine Meßblende (7) zu installieren, die über eine Auswertungseinrichtung (8) mit der Steuerung (6) verbunden ist. Alternativ oder ergänzend zu einer Messung einer Druckamplitude des Atemgases ist es auch möglich, im Hinblick auf oszillierende Anteile eine Phasenmessung durchzuführen. Die jeweiligen Sensoren (5) für Druckmessungen und Phasenmessungen können ebenfalls über Auswertungseinrichtungen (9,10) an die Steuerung (6) angeschlossen sein.

Zur Durchführung einer Mustererkennung ist mindestens einer der Sensoren (5) mit einem Analysator (11) verbunden, der an die Steuerung (6) angeschlossen ist. Der Analysator (11) wirkt mit einem Speicher (12) zur Bereitstellung von Vergleichsmustern sowie mit einem Klassifizierer (13) zur Auswertungsunterstützung zusammen. Insbesondere ist daran gedacht, die Steuerung (6), den Analysator (11), den Speicher (12) sowie den Klassifizierer (13) mindestens teilweise als Ablaufprogramm einer geeigneten Datenverarbeitungsanlage zu realisieren. Ebenfalls ist es möglich, die Auswertungseinrichtungen (8,9,10) mindestens teilweise in Form von Software zu realisieren.

Eine Verwendung der Beatmungseinrichtung mit Mustererkennung ist insbesondere im Bereich der CPAP-Beatmung eines Patienten (1) möglich. Bei einer derartigen Beatmung kann eine rechtzeitige Erkennung einer Schlafapnoe dadurch unterstützt werden, daß dem von der Druckgasquelle (4) erzeugten Volumenstrom ein mit geringer Amplitude oszillierender Volumenstrom überlagert wird, der von einer separaten Pumpeinrichtung (14) erzeugt werden kann. Aus einer Auswertung eines mit der zugeordneten Frequenz oszillierenden Antwortsignals kann auf den jeweiligen Atmungszustand des Patienten rückgeschlossen werden.

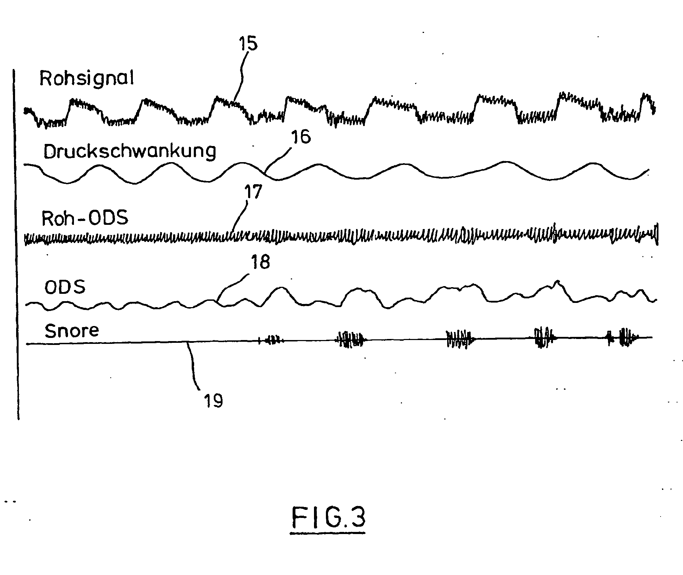

Fig. 3 zeigt typische Signalverläufe bei der Anwendung einer Mustererkennung. Der Verlauf (15) stellt hierbei das Rohsignal dar, der Verlauf (16) entspricht der aus den Einatmungs- und Ausatmungsvorgängen resultierenden Druckschwankung. Unter Berücksichtigung des Verlaufes (16) kann aus dem Verlauf (15) des Rohsignals ein Verlauf (17) erzeugt werden, der einem Roh-ODS entspricht. Nach einer weiteren Filterung kann hieraus ein Verlauf (18) extrahiert werden, der dem ODS entspricht. Über eine weitere Auswertung kann ein Verlauf (19) ermittelt werden, der einem SchnarchSignal entspricht.

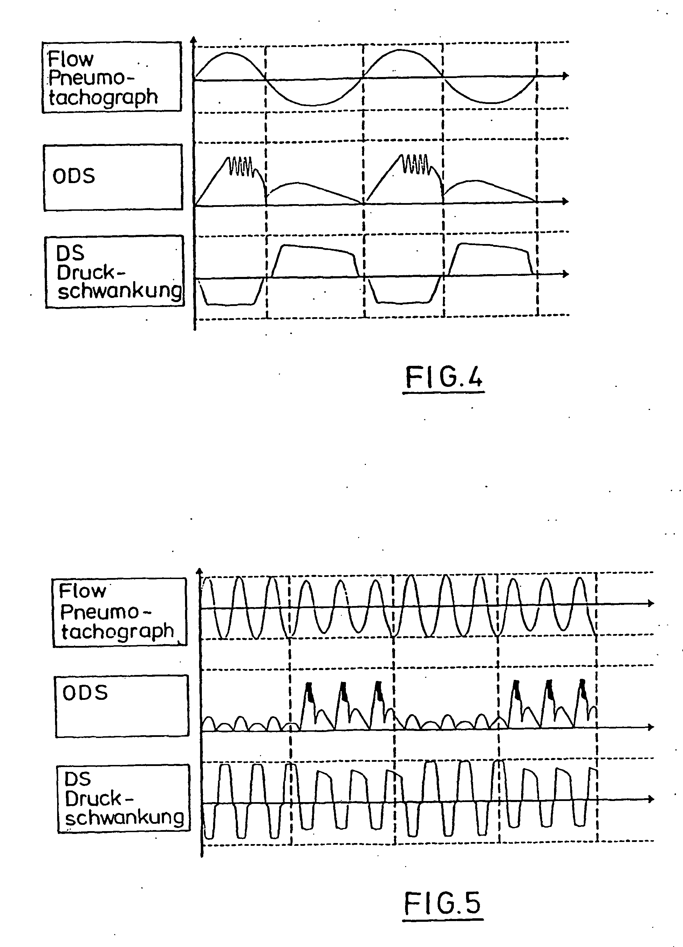

Das Prinzip der Mustererkennung wird anhand von Fig. 4 und Fig. 5 weiter erläutert, wobei typische Signalverläufe bei einem Schnarchen erläutert werden. Fig. 4 zeigt eine Mustererkennung im Hinblick auf Formmerkmale. Es sind das Ausgangssignal des FLOW-Pneumotachographen, das ermittelte ODS-Signal sowie die Druckschwankung (DS) dargestellt.

Fig. 5 zeigt eine Signalauswertung im Hinblick auf Zeitmerkmale. Auch hier sind wieder das Ausgangssignal des FLOW-Pneumotachographen, das ODS-Signal sowie die DS-Druckschwankung dargestellt.

Eine akute Symptomatik der Schlafapnoe tritt in der Regel lediglich kurzfristig auf. Typischerweise kommt es während des Schlafs des Patienten zu Gewebeerschlaffungen im Halsbereich in einer Umgebung der Luftwege, die zu einem Verschluß der Luftwege führen. Die aus den Gewebeerschlaffungen resultierende Atemnot führt zu häufigen Aufweckreaktionen, so daß der benötigte Tiefschlaf nicht erreicht wird. Dies hat extreme Tagesmüdigkeit und negative Auswirkungen auf das Herz-Kreislauf-System zur Folge.

Zur Vermeidung von Erstickungsanfällen wird dem Patienten während des Schlafs durch geeignete Beatmungsmasken ein Überdruck in den Bereich der Atemwege zugeführt, der das Gewebe abstützt und einem Zusammenfallen des Gewebes mit anschließendem Verschluß der Luftwege entgegenwirkt. Zur Unterstützung eines verbesserten Atmungskomforts ist es auch möglich, während des Einatmens ein höheres Druckniveau und während des Ausatmens ein relativ zu diesem höheren Druckniveau geringeres Druckniveau bereitzustellen, da der Patient während der Ausatmungstätigkeit selbst zum resultierenden Gesamtdruck beiträgt.

Zur Bereitstellung eines Beatmungsdruckes, der dafür geeignet ist, bei einem Auftreten einer akuten Symptomatik der Schlafapnoe eine Beeinträchtigung der Patientenatmung zu verhindern und der nur dann aufgebaut wird, wenn meßtechnisch ermittelt wurde, daß kurzfristig ein derartiger Zustand auftreten könnte, ist es vorteilhaft, daß dem für die Atmungsunterstützung des Patienten bereitgestellten Volumenstrom an Atemgas ein mit definierter Frequenz oszillierender Volumenstrom überlagert wird. In Abhängigkeit von den jeweiligen Luftwegsgeometrien des Patienten korrespondiert zu diesem oszillierenden Volumenstrom eine Druckschwingung, deren Betragsgröße vom Atemwegswiderstand des Patienten abhängig ist.

Bei der Durchführung dieses Steuerverfahrens wird zunächst in einem Ausgangszustand ein Basiswert für die Amplitude des sich ergebenden oszillatorischen Druckverlaufes ermittelt, der einem initialen Normalzustand der Atemwege des Patienten entspricht. Um Änderungen des Basiswertes im Verlauf der Therapie gerecht zu werden, wird anschließend der Basiswert fortlaufend an die aktuellen Verhältnisse angepaßt.

Die Frequenz und der Volumenhub des oszillierenden Volumenstromes werden so gewählt, daß für den Patienten keine individuell erfaßbare Auswirkung zu bemerken ist. Typischerweise liegt die Frequenz des oszillatorischen Volumenstromes im Bereich von 5 Hz bis 20 Hz, in der Regel bei 20 Hz und die Menge dieses Volumenstromes beträgt typischerweise lediglich etwa 1 Milliliter Atemluft, die dem Grundvolumenstrom an Atemgas oszillatorisch überlagert wird.

Aufgrund der bekannten Frequenz des oszillierenden Signals kann eine selektive Auswertung der resultierenden Druckamplitude meßtechnisch äußerst einfach durchgeführt werden.

Für den Fall, daß meßtechnisch ein Ansteigen der oszillatorischen Druckamplitude festgestellt wird, wird durch die Gerätesteuerung der eigentliche Atmungsdruck erhöht und hierdurch eine verstärkte Abstützung des Gewebes der Atemwege des Patienten erreicht. Eine Erhöhung der Druckamplitude des oszillierenden Drucksignals signalisiert hierbei, daß eine beginnende Verengung der Atemwege auftritt. Bezugswert ist jeweils der Basiswert für die oszillatorische Druckamplitude, die im Normalzustand des Patienten ermittelt wurde.

Die Erhöhung des Beatmungsdruckes wird mit entsprechender Zeitverzögerung schrittweise solange fortgesetzt, bis die oszillatorische Druckamplitude wieder abnimmt und die Gefahr einer akuten Symptomatik vorbei ist.

Nach Erreichen dieses Zustandes wird der eigentliche Beatmungsdruck ebenfalls wieder abgesenkt.

Ergänzend zur Mustererkennung ist es somit möglich, durch Auswertung eines Testsignals das eigentliche Beatmungsgerät bedarfsabhängig zu steuern. Es wird hierdurch im technischen Sinn kein eigentliches Beatmungsgerät mehr bereitgestellt, sondern es erfolgt lediglich bedarfsabhängig eine Unterstützung der Patientenatmung.

Für die Durchführung der Mustererkennung können neben den eben bereits erwähnten Signalen beispielsweise auch Geräuschsignale ausgewertet werden. Eine Auswertung kann sich hierbei unter anderem selektiv auf niederfrequente Anteile oder hochfrequente Anteile beziehen. Eine derartige Auswertung bietet sich beispielsweise bei der Analyse von Schnarchgeräuschen gemäß Fig. 4 und Fig. 5 an. Ebenfalls ist daran gedacht, eine Auswertung selektiv schmalbandig oder breitbandig durchzuführen.

Aufgrund der Ergebnisse der Mustererkennung kann aber nicht nur auf einen jeweiligen Beatmungszustandes des Patienten rückgeschlossen werden. Darüber hinaus ist es möglich, aus bestimmten Mustern auch auf den Gerätezustand des Beatmungsgerätes zurückzuschließen und rechtzeitig Funktionsstörungen zu erkennen. Einer Gefährdung des Patienten durch einen Geräteausfall kann hierdurch aufgrund einer rechtzeitigen Signalisierung eines sich anbahnenden Defektes entgegengewirkt werden. Ebenfalls ist es auf diese Weise möglich, ein Nachlassen der Leistungsfähigkeit des Gerätes rechtzeitig zu erkennen.

Generell können je nach Art des ausgewählten Signals unterschiedliche Rückschlüsse auf bestimmte Parameter gewonnen werden. Aus dem ODS-Signal kann ein Rückschluß auf die respiratorische Impedanz gezogen werden. Ein aus dem Signal der Druckschwankung abgeleitetes Signal kann beispielsweise nach einer Herausfilterung des Gleichanteiles für Rückschlüsse auf den CPAP-Druck herangezogen werden. Mit dem betreffenden Gleichanteil erfolgt ein Rückschluß auf den Atemfluß. Wird das Signal einem Bandpaß im Bereich von 30 Hz bis 300 Hz unterworfen, so kann ein Rückschluß auf ein Schnarchsignal erfolgen. Bei einer Auswertung von elektrischen Parametern, beispielsweise der Motorspannung des Gebläses, kann ein Rückschluß auf den Atemfluß erfolgen, eine Analyse des Gerätezustandes kann unter anderem auch Informationen über mögliche Leckagen im Bereich des Gerätes selbst oder im Bereich der Schlauchverbindungen liefern.

Eine Durchführung der Mustererkennung zur Analyse von Formmerkmalen greift auf eine Parameterextraktion in Bezug auf Pegel- und Amplitudenwerte, Zeitabstände, Einhüllende, Nulldurchgänge oder Steigungen zurück. Bei einer Analyse im Hinblick auf Zeitmerkmale wird beispielsweise bei einer Parameterextraktion auf Periodizitäten und Frequenzen zurückgegriffen.

Die vorstehende Erläuterung der Ermittlung der Atemfrequenz dient als typisches Beispiel für die Auswertung von Zeit- und Amplitudenmerkmalen. Die Anwendung des vorgeschlagenen Verfahrens sowie der Vorrichtung sind jedoch nicht auf dieses Beispiel beschränkt.

Während der Durchführung der Beatmungstherapie erfolgt im Allgemeinen eine Erfassung und Speicherung von Meßgrößen, die individuell auf den therapierten Patienten bezogen sind. Die Kenntnis dieser Meßgrößen kann deshalb bei der Durchführung einer Druckregelung in nachfolgenden Therapien mit einbezogen werden.

Ebenfalls ist daran gedacht, eine Erfassung und Speicherung von Meßgrößen durchzuführen, die auf bestimmte Patientenkollektive bezogen sind. Dies hat zur Folge, daß die Kenntnis dieser Meßgrößen bei der Druckregelung eines individuellen Patienten genutzt werden kann, nachdem die Zugehörigkeit dieses individuellen Patienten zu einem Patientenkollektiv ermittelt worden ist.

Die bereits erläuterte Schnarcherkennung kann beispielsweise wie nachfolgend erläutert durchgeführt werden.

Durch eine Analog - Digital - Wandlung des Maskendruckes mit einer Abtastfrequenz von 500 Hz und einer anschließender digitalen Bandpaßfilterung im Frequenzbereich von 65 Hz - 190 Hz, wird die Druckschwankung innerhalb des Durchlaßbereiches des Filters als Schnarchen interpretiert.

Die Ausgangswerte des Filters werden einer Atemphase, Inspiration und / oder Exspiration, zugeordnet. Um die Ausgangswerte von Strömungsgeräuschen und anderen Artefakten unterscheiden zu können, wird erst bei der Überschreitung eines Mindestpegels auf ein gültiges echtes Schnarchen geschlossen.

Nachfolgend wird die Dauer des Schnarchens im Verhältnis zur Atemphase, Inspiration / Exspiration, ermittelt. Übersteigt dieser Quotient einen Mindestwert, gilt das Schnarchen, abhängig von der Atemphase, als therapiewürdig.

Die Ergebnisse vor Werteübergabe an die Drucksteuerung sind:
a) Schnarchen ja / nein
b) Schnarchen inspiratorisch / exspiratorisch
c) Quotient Inspirationszeit / Schnarchzeit

Eine Atmungserkennung kann wie nachfolgend erläutert durchgeführt werden.

Durch eine Analog - Digital - Wandlung des Maskendruckes mit einer Abtastfrequenz von 25 Hz und einer anschließender digitalen Bandpaßfilterung im Frequenzbereich von 0,1 Hz - 0,6 Hz wird die Druckschwankung innerhalb des Durchlaßbereiches des Filters als Atmung interpretiert.

Die Ausgangswerte des digitalen Filters nehmen während der Inspiration negative, während der Exspiration positive Werte an. Nach Ermittlung von Inspirationszeit und Exspirationszeit wird die momentane Atemfrequenz berechnet. Um die Ausgangswerte von Strömungsgeräuschen und anderen Artefakten unterscheiden zu können, wird erst bei Überschreitung eines Mindestpegels auf eine gültige echte Atmung geschlossen.

Die Ergebnisse vor Werteübergabe an das Atemhistogramm sind:
a) Atmung erkannt
b) Gültige Atemfrequenz

Ein Atemhistogramm kann wie nachfolgend beschrieben ermittelt werden.

Das Atemhistogramm beinhaltet die letzten 20 als gültig erkannten Atemfrequenzen. Die von der Atmungserkennung als gültig erkannte Atemfrequenz wird dem Atemhistogramm als neuer zwanzigster Wert übergeben. Die bisherigen Atemfrequenzwerte werden um einen Platz im Atemhistogramm verschoben, so daß immer 20 Atemfrequenzwerte im Atemhistogramm stehen.

Innerhalb des Atemhistogramms wird die am häufigsten auftretende Frequenz ermittelt. Übersteigt die Häufigkeit dieser Frequenz einen Mindestwert, dann gilt diese Frequenz als die häufigste. Diese häufigste Atemfrequenz gilt als Ausschlußkriterium für die nachfolgende nächste Atemfrequenz. Weicht die aktuell als gültig erkannte Atemfrequenz um einen Mindestwert von der häufigsten Atemfrequenz ab, wird auf ein Atemartefakt geschlossen.

Durch die Ermittlung der häufigsten Atemfrequenz wird ein stabiler Zustand der Atmung und damit des Patienten angenommen.

Die Ergebnisse der Auswertung des Atemhistogramms sind:
a) Stabiler Atmungszustand
b) Atemartefakt ja / nein

Die Initialisierungsphase dient zur Definition eines Grundzustandes des Gerätes. Die erste Minute nach dem Einschalten des Gerätes wird zur Initialisierung und Kommunikation der beiden eingesetzten Geräteteile verwendet. Die Initialisierung des ODS Referenzwertes wird nach einer Zeit von 2 Minuten und dem Erkennen einer stabilen Atemfrequenz durch die Auswertung des Atemhistogramms abgeschlossen.

Eine Drucksteuerung kann aufgrund von ODS-Anstiegen durchgeführt werden. Übersteigt der aktuelle ODS Wert den während der Initialisierung ermittelten ODS - Referenzwert um mindestens 60 %, wird bei gleichzeitig stabilem Zustand der Atmung, erkannter Atmung, gültiger Atemfrequenz und erkannter Inspiration der Druckwert mit einer Steigung von 0,2 mbar/sek. angehoben.

Übersteigt der aktuelle ODS Wert den während der Initialisierung ermittelten ODS - Referenzwert um mindestens 60 % und trifft gleichzeitig eines der aus der Atmungsanalyse gewonnenen Kriterien nicht zu, so wird der Druck nicht angehoben.

Eine Drucksteuerung aufgrund von Schnarchen kann beispielsweise wie nachfolgend erläutert durchgeführt werden.

Übersteigt der aktuelle ODS Wert den während der Initialisierung ermittelten ODS - Referenzwert um mindestens 40 %, wird bei gleichzeitig stabilem Zustand der Atmung, erkannter Atmung, gültiger Atemfrequenz, erkannter Inspiration und Überschreitung des Mindestwertes des Quotienten aus Inspirationszeit / Schnarchzeit, der Druckwert mit einer Steigung von 0,1 mbar/sek. angehoben.

Eine weitere Variante bei der Durchführung des Verfahrens sowie bei der konstruktiven Realisierung der Vorrichtung besteht darin, daß Druckschwankungen mit einer Frequenz erzeugt werden, die eine Anregung der Muskulatur zur Folge hat. Eine derartige Anregung der Muskulatur durch Druckschwankungen kann als "Forced Ozcillation technic" (FOT) bezeichnet werden. Es zeigt sich, daß durch derartige Druckschwankungen eine Aktivierung von Rezeptoren hervorgerufen wird.

Derartige Rezeptoren sind im Bereich der Nase, des Rachens und der oberen Atemwege eines Patienten angeordnet. Im Hinblick auf die Gerätesteuerung ist deshalb insbesondere daran gedacht, bei einer Erkennung eines Atemwegverschlusses durch die erläuterten Möglichkeiten der Mustererkennung eine Gerätesteuerung derart durchzuführen, daß in ihrer Frequenz und ihrer Amplitude vorgegebene Druckschwingungen generiert werden, die zu einer Aktivierung der Rezeptoren führen, wobei die Druckschwingungen typischerweise über eine Nasalmaske zugeführt werden. Die Frequenzen liegen bevorzugt in einem Bereich oberhalb von 0,1 Hz. Insbesondere ist daran gedacht, über das Beatmungsgerät sowohl die Frequenz als auch die Amplitude der Druckschwankungen unabhängig voneinander zu variieren. Darüber hinaus ist es möglich, adaptiv aufgrund einer Auswertung der Mustererkennungen die Dauer der Applikation der Druckschwingung in Abhängigkeit vom jeweils erfaßten Anwendungszustand zu variieren.

Gerätetechnisch können als Oszillationsgeneratoren zur Bereitstellung der Druckschwankungen Lautsprecher, modifizierte Membranpumpen oder andere Aktoren eingesetzt werden.

Bei der Durchführung der Signalauswertung ist insbesondere auch an eine Trennung von Inspirations- und Exspirationssignalen gedacht. Dies bedeutet, daß die ODS-Signale für beide Phasen separat ermittelt werden und daß es hierdurch insbesondere möglich ist, ein Anheben und Absenken des Beatmungsdruckes nur in Abhängigkeit von den Informationen der inspiratorischen Druckmeßwerte durchzuführen. Alternativ ist es auch möglich, eine Veränderung des Druckes sowohl in Abhängigkeit von den inspiratorischen als auch in Abhängigkeit von den exspiratorischen Werten durchzuführen, die Beeinflussung des Druckes jedoch unterschiedlich durchzuführen, je nachdem ob inspiratorische oder exspiratorische Eingangssignale vorliegen.

## Patentansprüche

1. Beatmungsgerät mit einer Vorrichtung zur Überwachung mindestens eines Beatmungsparameters bei des Atemgasversorgung eines Patienten, die mindestens einen Sensor (5) zur Erfassung eines zeitlichen Verlaufes des Beatmungsparameters aufweist, der im Bereich einer Luftzuführung angeordnet ist, die eine verbindungseinrichtung umfaßt, wobei der Sensor (5) mit einem Analysator (11) verbunden ist, der eine Mustererkennung durchführt und der an eine Steuerung (6) zur Veränderung mindestens eines Beatmungsparameters angeschlossen ist, **dadurch gekennzeichnet daß** der Analysator (11) sowohl zur Auswertung eines Beatmungsdruckes als auch zur Auswertung eines Beatmungsflows ausgebildet ist und bei der der Analysator (11) zusätzlich zur Auswertung des Beatmungsdruckes und des Beatmungsflows zur Auswertung einer aus einem ODS-Signal ermittelten respiratorischen Impedanz ausgebildet ist und daß der Analysator (11) mit einem Klassifizierer (13) zur Auswertungsunterstützung gekoppelt ist.

2. Vorrichtung nach Anspruch 1, **dadurch gekennzeichnet, daß** der Analysator (11) mit einem Speicher (12) zur Bereitstellung von vergleichsmustern gekoppelt ist.

3. Vorrichtung nach einem der Ansprüche 1 oder 2, **dadurch gekennzeichnet, daß** der Analysator (11) einen Analysator für den Signalverlauf aufweist.

4. Vorrichtung nach einem der Ansprüche 1 bis 3, **dadurch gekennzeichnet, daß** der Analysator (11) einen Analysator für die Signalform aufweist.

5. Vorrichtung nach einem der Ansprüche 1 bis 4, **dadurch gekennzeichnet, daß** der Analysator (11) zur Mustererkennung als Teil eines Beatmungsgerätes zur Durchführung einer CPAP-Beatmung ausgebildet ist.

6. Vorrichtung nach einem der Ansprüche 1 bis 5, **dadurch gekennzeichnet, daß** der Analysator (11) einen Speicher für mindestens eine Meßgröße aufweist.

7. Vorrichtung nach einem der Ansprüche 1 bis 6, **dadurch gekennzeichnet, daß** die Steuerung (6) zur Durchführung einer Druckveränderung in Abhängigkeit von der abgespeicherten Meßgröße ein Adaptionselement aufweist.

8. Vorrichtung nach einem der Ansprüche 1 bis 7, **dadurch gekennzeichnet, daß** der Analysator (11) mindestens einen Speicher für Daten eines Patientenkollektives aufweist.

9. Vorrichtung nach einem der Ansprüche 1 bis 8, **dadurch gekennzeichnet, daß** die Steuerung (6) zur Durchführung einer Druckveränderung in Abhängigkeit von den abgespeicherten Informationen des Patientenkollektives ein Adaptionselement aufweist.

10. Vorrichtung nach einem der Ansprüche 1 bis 9, **dadurch gekennzeichnet, daß** der Analysator (11) mit einem neuronalen Netz versehen ist.

11. Vorrichtung nach einem der Ansprüche 1 bis 10, **dadurch gekennzeichnet, daß** der Analysator (11) mit einer Fuzzy-Logik versehen ist.

12. Vorrichtung nach einem der Ansprüche 1 bis 11, **dadurch gekennzeichnet, daß** der Sensor (5) an mindestens ein Abtastelement angeschlossen ist.

13. Vorrichtung nach einem der Ansprüche 1 bis 12, **dadurch gekennzeichnet, daß** das Abtastelement mit einem Bandpaß-Filter verbunden ist.

14. Vorrichtung nach Anspruch 13, **dadurch gekennzeichnet, daß** der Bandpaß-Filter als ein digitaler Filter ausgebildet ist.

15. Vorrichtung nach einem der Ansprüche 1 bis 14, **dadurch gekennzeichnet, daß** die Steuerung (6) an einen Generator zur Erzeugung von rezeptorstimulierenden Drucksignalen angeschlossen ist.

16. Vorrichtung nach einem der Ansprüche 1 bis 15, **dadurch gekennzeichnet, daß** der Analysator (11) separate Auswertungen für inspiratorische und für exspiratorische Druckmeßwerte aufweist.

17. Vorrichtung nach Anspruch 16, **dadurch gekennzeichnet, daß** für die inspiratorischen und die exspiratorischen Druckmeßwerte unterschiedliche Drucksteuerungscharakteristiken vorgesehen sind.

## Claims

1. Breathing device with a device for monitoring at least one breathing parameter in the case of the respiratory gas supply of a patient, which device has at least one sensor (5) for detecting a temporal progression of the breathing parameter, which sensor is arranged in the area of an air supply, which comprises a connecting device, the sensor (5) being connected to an analyzer (11), which executes a pattern detection and which is connected to a control (6) for changing at least one breathing parameter, **characterized in that** the analyzer (11) is formed both to evaluate a breathing pressure and to evaluate a breathing flow and in which the analyzer (11) in addition to evaluation of the breathing pressure and the breathing flow is formed to evaluate a respiratory impedance determined from an ODS signal and that the analyzer (11) is connected to a classifier (13) for evaluation support.

2. Device according to claim 1, **characterized in that** the analyzer (11) is connected to a storage device (12) for supplying comparison patterns.

3. Device according to one of claims 1 or 2, **characterized in that** the analyzer (11) has an analyzer for the signal progression.

4. Device according to one of claims 1 to 3, **characterized in that** the analyzer (11) has an analyzer for the signal shape.

5. Device according to one of claims 1 to 4, **characterized in that** the analyzer (11) is formed for pattern detection as part of a breathing apparatus for executing CPAP breathing.

6. Device according to one of claims 1 to 5, **characterized in that** the analyzer (11) has a storage device for at least one measured quantity.

7. Device according to one of claims 1 to 6, **characterized in that** the control (6) for executing a pressure change as a function of the stored measured quantity has an adaptation element.

8. Device according to one of claims 1 to 7, **characterized in that** the analyzer (11) has at least one storage device for data of a patient collective.

9. Device according to one of claims 1 to 8, **characterized in that** the control (6) for executing a pressure change as a function of the stored information of the patient collective has an adaptation element.

10. Device according to one of claims 1 to 9, **characterized in that** the analyzer (11) is provided with a neural network.

11. Device according to one of claims 1 to 10, **characterized in that** the analyzer (11) is provided with a fuzzy logic.

12. Device according to one of claims 1 to 11, **characterized in that** the sensor (5) is connected to at least one scanning element.

13. Device according to one of claims 1 to 12, **characterized in that** the scanning element is connected to a band-pass filter.

14. Device according to claim 13, **characterized in that** the band-pass filter is formed as a digital filter.

15. Device according to one of claims 1 to 14, **characterized in that** the control (6) is connected to a generator for generating receptor-stimulating pressure signals.

16. Device according to one of claims 1 to 15, **characterized in that** the analyzer (11) has separate evaluations for inspiration and for expiration pressure measured values.

17. Device according to claim 16, **characterized in that** different pressure control characteristics are provided for the inspiration and expiration pressure measured values.

## Revendications

1. Appareil de respiration artificielle avec un dispositif de surveillance d'au moins un paramètre de la respiration lors de l'alimentation en gaz respiré d'un patient, qui présente au moins un capteur (5) pour détecter un développement temporel du paramètre de la respiration, qui est disposé au niveau d'une amenée d'air, qui comprend un dispositif de liaison, où le capteur (5) est relié à un analyseur (11), qui réalise une identification d'échantillon et qui est raccordé à un réglage (6) pour la modification d'au moins un paramètre de la respiration, **caractérisé en ce que** l'analyseur (11) est formé non seulement pour évaluer une pression de respiration, mais également pour évaluer un débit de respiration, et **en ce que** l'analyseur (11) est formé en outre pour l'évaluation de la pression de respiration et du débit de respiration, pour évaluer une impédance respiratoire déterminée à partir d'un signal ODS et **en ce que** l'analyseur (11) est couplé à un classeur (13) pour soutenir l'évaluation.

2. Dispositif selon la revendication 1, **caractérisé en ce que** l'analyseur (11) est couplé à une mémoire (12) pour la préparation d'échantillon de comparaison.

3. Dispositif selon l'une des revendications 1 ou 2, **caractérisé en ce que** l'analyseur (11) présente un analyseur du développement du signal.

4. Dispositif selon l'une des revendications 1 à 3, **caractérisé en ce que** l'analyseur (11) présente un analyseur de la forme du signal.

5. Dispositif selon l'une des revendications 1 à 4, **caractérisé en ce que** l'analyseur (11) est formé pour une identification d'échantillon en tant que partie d'un appareil de respiration artificielle, pour réaliser une respiration CPAP.

6. Dispositif selon l'une des revendications 1 à 5, **caractérisé en ce que** l'analyseur (11) présente une mémoire pour au moins une grandeur mesurée.

7. Dispositif selon l'une des revendications 1 à 6, **caractérisé en ce que** le réglage (6) pour réaliser une modification de pression en fonction de la grandeur mesurée mémorisée présente un élément d'adaptation.

8. Dispositif selon l'une des revendications 1 à 7, **caractérisé en ce que** l'analyseur (11) présente au moins une mémoire pour les données d'un ensemble de patients.

9. Dispositif selon l'une des revendications 1 à 8, **caractérisé en ce que** le réglage (6) pour réaliser une modification de pression en fonction des informations mémorisées présente un élément d'adaptation.

10. Dispositif selon l'une des revendications 1 à 9, **caractérisé en ce que** l'analyseur (11) est muni d'un réseau neuronal.

11. Dispositif selon l'une des revendications 1 à 10, **caractérisé en ce que** l'analyseur (11) est muni d'un Fuzzy Logic.

12. Dispositif selon l'une des revendications 1 à 11, **caractérisé en ce que** le capteur (5) est raccordé à au moins un élément de balayage.

13. Dispositif selon l'une des revendications 1 à 12, **caractérisé en ce que** l'élément de balayage est relié à un filtre passe-bande.

14. Dispositif selon la revendication 13, **caractérisé en ce que** le filtre passe-bande est formé comme un filtre digital.

15. Dispositif selon l'une des revendications 1 à 14, **caractérisé en ce que** le réglage (6) est raccordé à un générateur pour produire des signaux de pression stimulant le récepteur.

16. Dispositif selon l'une des revendications 1 à 15, **caractérisé en ce que** l'analyseur (11) présente des évaluations séparées pour les valeurs de mesure de pressions inspiratoire et expiratoire.

17. Dispositif selon la revendication 16, **caractérisé en ce que** pour les valeurs de mesure de pressions inspiratoire et expiratoire, différentes caractéristiques de réglage de pression sont prévues.
